(19) Europäisches Patentamt
European Patent Office
Office européen des brevets

(11) Publication number: **0 071 999**
**B1**

(12) **EUROPEAN PATENT SPECIFICATION**

(45) Date of publication of patent specification: **26.03.86**

(21) Application number: **82107140.4**

(22) Date of filing: **06.08.82**

(51) Int. Cl.[4]: **C 12 N 5/02,** C 12 P 7/66 // C12R1/91, C12P17/16, C12P17/18

# (54) Method for producing secondary metabolites of plants.

(30) Priority: **11.08.81 JP 124764/81**
**11.08.81 JP 124765/81**
**11.08.81 JP 124766/81**
**11.08.81 JP 124767/81**
**11.08.81 JP 124768/81**
**11.08.81 JP 124769/81**

(43) Date of publication of application:
**16.02.83 Bulletin 83/07**

(45) Publication of the grant of the patent:
**26.03.86 Bulletin 86/13**

(84) Designated Contracting States:
**CH DE FR GB LI**

(56) References cited:
**EP-A-0 007 244**
**EP-A-0 022 434**

**CHEMICAL ABSTRACTS, vol. 93, no. 15, 13th October 1980, page 414, no. 146587p, Columbus, Ohio, USA A. KATO et al.: "Biomass production of tobacco cells. V. Biomass production of nitrogen-reduced tobacco cells in two-stage continuous culture"**

(73) Proprietor: **MITSUI PETROCHEMICAL INDUSTRIES, LTD.**
**2-5, Kasumigaseki 3-chome Chiyoda-ku**
**Tokyo 100 (JP)**

(72) Inventor: **Hara, Yasuhiro**
**3-8, Misono 1-chome**
**Ootake-shi Hiroshima (JP)**
Inventor: **Suga, Chuzo**
**2-2, Misono 1-chome**
**Ootake-shi Hiroshima (JP)**

(74) Representative: **Brehm, Hans-Peter, Dr. Dipl.-Chem. et al**
**Patentanwälte Tischer, Kern & Brehm Albert-Rosshaupter-Strasse 65**
**D-8000 München 70 (DE)**

**EP 0 071 999 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

## Description

The present invention relates to a method for producing secondary metabolites of plants by culturing in a first liquid medium, the composition thereof favours the growth of said plants and comprises inorganic substances and carbon sources as the essential constituents and further containing at least one of phytohormones, vitamines and amino acids, and further culturing said cells obtained from the first medium in a second liquid medium, the composition thereof favours the production of the secondary metabolites.

A method of said type is known from European Patent Publication 7 244. The known method comprises cultivating a strain of *Nicotiana tabacum* or *Nicotiana rustica* in suspension culture under sub-maximal growth conditions which do not inhibit nicotin production. Suspension cultures are obtained by transferring several grams or soft, loosely packed callus tissue into a liquid medium, followed by continous gentle agitation. Initially, cells are grown under maximal growth conditions in a first vessel into which fresh medium is continously or intermittently introduced. The cells in the substantially exhausted medium are decanted and are allowed to age. The aged cells are treated in a third vessel or seris of vessels, with a medium in which the cells grow, in a second growth phase, under sub-maximal growth conditions. There are a number of ways in which conditions can be controlled in order to achieve sub-maximal growth when it is desired to start nicotine production. One is to introduce a growth inhibitor and another is to limit growth nutrients, i.e. to reduce or omit nutrients from the medium which should normally contain assimilable source of carbon (such as sucrose or molasses) and nitrogen and other nutrients and vitamines. It is said, a particularly preferred method of inhibiting growth and thereby increasing nicotine production is to control the hormone level.

Obviously, said two-stage method is not limited to the production of nicotine. The European Patent Publication 22 434 discloses the production of other secondary metabolites such as anthraquinone derivates or indole alkaloides like ajmalicine using immobilized cells. An increased yield of secondary metabolites can be induced by placing the immobilzed cells in a nutrient so modified that the growth of cells is inhibited. By hormone-limiting conditions at the production an inhibited growth thus results in a drastically increased yield. Further it is said, phosphate is an important nutrient for growing plant cells. By phosphate, and also sulphate, limitation the growth of cells is inhibited, which results in an increased synthesis of secondary metabolites. In this case, too, the availability of nitrogen and carbon sources under growth-limiting conditions would seem to lead to an increased rate of synthesis of alkaloids and other secondary products.

A typical example of secondary metabolites which may be obtained according to the method of the present invention concerns naphthoquinone derivates such as shikonin (R = —OH) as represented in the formula below

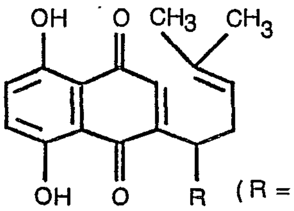

( R = $-OH$, $-OCOCH_3$, etc.)

Shikonin is produced in the roots of *Lithospermum erythrorhizon* Sieb. et Zucc. (*Boraginaceae)* and has been conventionally used as a material of the Chinese herb machines being called "Shikon" (literally meaning "purple root"). That is, the ointments obtained by extracting shikonin and other substances from such plant roots with sesame or other oil are called "Shi Un Ko" and known to possess anti-inflammatory activity and stimulate the formation of granulation tissue, among other medical actions. Thus, they are used as topical medications for dermal deseases, wounds, burns, hemorrhides, etc.

However, the amount of the effective substances such as shikonin which can be extracted from the plant roots is very slight. The cultivation of *L. erythrorhizon* takes a long time and the harvesting depends on the natural environments and weather conditions. Thus, the stable supply of shikonin is difficult.

Incidentally, experiences of proliferation of *L. erythrorhizon* plants by using the cell culture method have been reported by M. Tabata, H. Mizukami, and others is "Phytochemistry" Vol. 13, page 927, "Pharmacological Journal" Vol. 95, page 1376, "Phytochemistry" Vol. 16, page 1183 and Vol. 17, page 95. These methods are quite advantageous because *L. erythrorhizon* plants may be proliferated steadily regardless of the season and the weather.

The object of the present invention is to adapt the above stated method to the production of further selected secondary metabolites and/or to increase the yield of said secondary metabolites.

Starting from the above stated method for producing secondary metabolites of plants, the present invention is characterized in that the composition of the second liquid medium differs, from the composition of the corresponding first liquid medium in at least two of the following concentrations (a) to (c), namely:

(a) the first medium contains inorganic nitrogen in an amount of 40 to 130 mM, and the second liquid medium contains inorganic nitrogen in a concentration of not more than 1/5 of the respective concentration in the first medium;

(b) the first liquid medium contains inorganic phosphorus in an amount of 0.8 to 2.4 mM, and the second liquid medium contains inorganic phosphorus in a concentration of not more than 1/5 of the respective concentration in the first medium;

(c) the first liquid medium, contains potassium

in an amount of 10 to 45 mM, and the second liquid medium contains potassium in a concentration of not more than 1/5 of the respective concentration in the first medium; and said composition differs concurrently in at least one of the following concentrations (d) to (i), namely:

(d) the first liquid medium contains copper ions in an amount of 0.015 to 0.15 µM, and the second liquid medium contains copper ions in at least the three times concentration of the respective concentration in the first medium;

(e) the first liquid medium contains manganese ions in an amount of 30 to 300 µM, iodine ions in an amount of 1 to 15 µM, and molybdate ions in an amount of 0.05 to 0.5 µM, and the second liquid medium contains at least one of manganese ions, iodine ions and molybdate ions in a concentration of not more than 1/10 of the respective concentration in the first medium;

(f) the first liquid medium contains vitamins in a total amount of 1 to 500 mg/liter, and the second liquid medium contains vitamins in a concentration of not more than 1/10 of the respective concentration in the first medium;

(g) the first liquid medium contains amino acids in a total amount of 0.1 to 100 mg/liter, and the second liquid medium contains amino acids in a concentration of not more than 1/100 of the respective concentration in the first medium;

(h) the first liquid medium contains cytokinins in a total amount of 0.01 to 10 µM, and the second liquid medium contains cytokinins in a concentration of not more than 1/100 of the respective concentration in the first medium;

(i) the first liquid medium contains sulfate ions in an amount of 0.5 to 3 mM, and the second liquid medium contains sulfate ions in at least the ten times concentration of the respective concentration in the first medium.

Preferably, said method is used to produce naphthochinon derivates from plants of the *Boraginaceae* family, of the *Juglandaceae* family or of the *Lythraceae* family. Even more preferred, said method is used to produce shikonin from *Lithospermum erythrorhizon.*

According to another preferred aspect, said method is used to produce isoquinoline alkaloides from plants of the *Ranunculaceae* family. Even more preferred, said method is used to produce berberine from *Coptis japonica.*

According to a further preferred aspect, said method is used to produce the secondary metabolites from plants of the *Solanaceae* family; for example, nicotine is produced from *Nicotiana tabacum.*

In the following the method of the present invention is explained in more detail taking account to preferred embodiments thereof.

The liquid medium employable in the first stage of the present invention (referred to as "liquid medium (A)") may be one of those conventionally used for the plant cultures, which contains indispensably inorganic substances and carbon sources, and additionally, at least one of phytohormones, vitamins and amino acids.

Among those inorganic substances, there may be mentioned nitrogen, phosphorus, potassium, calcium, magnesium, sulfur, iron, manganese, zinc, boron, copper, molybdenum, chlorine, sodium, iodine, cobalt, etc. Typically, there may be illustrated potassium nitrate, sodium nitrate, calcium nitrate, potassium dihydrogen phosphate, disodium hydrogen phosphate, potassium chloride, calcium chloride, magnesium sulfate, sodium sulfate, iron (II) sulfate, iron (III) sulfate, manganese sulfate, zinc sulfate, boric acid, copper sulfate, sodium molybdate, molybdenum trioxide, potassium iodide, cobalt chloride, and the like.

As the carbon sources, carbon hydrates such as sucrose, their various derivatives, organic acids, such as fatty acids, primary alcohols, such as ethanol, and others may be illustrated.

The phytohormones or plant hormones may include, for example, auxins such as indoleacetic acid (IAA), p-chlorophenoxyisobutyric acid, 2,4-dichlorophenoxyacetic acid (2,4-D) and the like; further cytokinins such as kinetin, 6-benzyladenine (BA), 6-(3-methyl-2-butenylamino) purine (2ip), zeatin, dihydrozeatin and the like.

The vitamins may include biotine, thiamine (vitamin $B_1$), pyridoxine (vitamin $B_6$), pantothenic acid, ascorbic acid (vitamin C), inositol, nicotinic acid, and the like.

The amino acids may include glycine, alanine, glutamine, cystein and the like.

The concentration of each substance in the liquid medium (A) may be varied in the following ranges. Usually, the medium is prepared by adding 40 to 130 mM of N, 0.8 to 2.4 mM of P, 10 to 45 mM of K, 0.015 to 0.15 µM of Cu, 30 to 300 µM of Mn, 1 to 15 µM of J, 0.05 to 0.5 µM of $MoO_4$, and 0.5 to 3 mM of $SO_4^{--}$; carbon sources about 1 g/liter to 30 g/liter; phytohormones about 0.01 µM to 10 µM, for example 0.01 to 10 µM cytokinin; vitamins about 1 mg/liter to about 500 mg/liter; and amino acids about 0.1 mg/liter to about 100 mg/liter.

The liquid medium (A) is preferably one of those suited to growth or proliferation of cells. Practical examples may include Linsmaier-Skoog's medium, Gamborg et al's B-5 medium, and modified media thereof.

The cells cultured in the liquid medium (A) in the first stage are separated from said medium, and are added to a modified liquid medium (B) in the second stage, and the culturing of said cells is continued.

In the composition of the modified liquid medium (B), the concentration of at least three substances contained in the liquid medium (A) should be substantially modified, that is, increased or decreased, as stated in more detail in the following.

The concentrations of at least two constituents of the group including inorganic nitrogen, inorganic phosphorus and potassium in the liquid medium (B) will be decreased to not more than

1/5 of the respective concentration in the first liquid medium (A). In addition, the concentration of at least one further constituent of the group including copper ions, manganese ions, iodine ions, molybdate ions, sulfate ions, vitamins, amino acids, and cytokinins, in the liquid medium (B) will be modified, namely will be decreased to not more than 1/10 of the respective concentration in the first liquid medium (A) in the case of manganese ions, iodine ions and molybdate ions; further said concentration is decreased to not more than 1/10 of the respective concentration in the first liquid medium (A) in the case of vitamins; and further, said concentration is decreased to not more than 1/100 of the respective concentration in the first liquid medium (A) in the case of amino acids and cytokinins; and further, said concentration will be increased to not less than three times the concentration of the first liquid medium (A) in the case of copper ions; and further, said concentration will be increased to not less than ten times of the respective concentration in the first liquid medium (A) in the case of sulfate ions.

Referring now in more detail to the culturing in the modified liquid medium in the second stage of the present invention, which is prepared by substantially varying the concentration of at least three constituents of the liquid medium (A).

(a), (b), (c) The concentration of at least two components of the group comprising inorganic nitrogen, inorganic phosphorus and potassium will be decreased. That is, the present invention provides a method of producing secondary metabolites of plants, whereby the cells of a plant are initially cultured (in the first stage) in a liquid medium for plant tissue culture, containing inorganic nitrogen in an amount of 40 to 130 mM, inorganic phosphorus in an amount of 0.8 to 2.4 mM and potassium compounds in an amount of 10 to 45 mM. Thereafter, said cells are transferred (in the second stage) in a modified liquid medium (B) and culturing is continued in said modified liquid medium, wherein the concentration of at least two components namely inorganic nitrogen and inorganic phosphorus, or inorganic nitrogen and potassium or inorganic phosphorus and potassium is descreased to 1/5 or even less of the respective concentration in the first medium.

Additionally and concurrently at least one of the following concentrations (d) to (i) is modified.

(d) The copper ion concentration in the second stage will be increased. That is, the concentration of copper ions used in the medium of the first stage ranges from 0.01 to 0.15 μM, and the second medium contains copper ions in at least the three times concentration of the respective concentration in the first medium. Preferably the copper concentration of the second medium amounts at least 0.45 μM, more preferred the copper concentration of the second medium is adjusted within a range of 0.2 μM to 25 μM. When the copper ion concentration is less than three times, the production rate of naphthroquinone derivates decreases, and if the concentration is over 25 μM, no significant changes are observed, but the productivity decreases slightly.

(e) The concentration of at least one constituent of the group comprising manganese ions, iodine ions and molybdate ions in the second medium is decreased. That is, the manganese concentration of the medium used in the first stage is kept within a range of 30 to 300 μM, the iodine concentration is kept within 1 to 15 μM, and the molybdate concentration is kept within 0.05 to 0.5 μM; and the second medium contains manganese ions, iodine ions and/or molybdate ions in a concentration of not more than 1/10 of the respective concentration in the first medium. Preferably the concentration of at least two of said constituents is decreased; even more preferred, the concentration of all of said three constituents will be decreased. More particularly, the concentrations of said three constituents are preferably maintained as low as possible within the conditions specified for the second medium. It is most preferred to use a second liquid medium which does not contain any of such elements Mn, J, $MoO_4$ at all. As a result, the production of naphthoquinone derivative in the cells may be further increased.

(f) The total concentration of the vitamins is decreased. That is, the total concentration of the vitamins in the medium used in the first stage is maintained within 1 to 500 mg/liter; and the second liquid medium contains vitamins in a total concentration of not more than 1/10 of the respective concentration in the first medium.

(g) The total concentration of the amino acids is decreased. That is, the total concentration of amino acids in the medium used in the first stage is maintained between 0.1 to 100 mg/liter, and the second liquid medium contains amino acids in a concentration of not more than 1/100 of the respective concentration in the first medium.

Preferably the concentration of at least one constituent of the group of vitamins comprising (s) inositol, (t) thiamine, (u) pyridoxine, (v) nicotonic acid, and (w) ascorbic acid, and the concentration of at least one constituent of the group of amino acids comprising (x) glycine, (y) L-cystine, and (z) L-glutamine is decreased. It is even more preferred to decrease the concentrations of at least two constituents in each category, and it is most preferred to decrease the concentrations of all said constituents from (s) to (z).

More particularly, the concentration of said constituents (s) to (z) are preferably maintained as low as possible within the conditions specified for the second liquid medium. It is most preferred to use a second liquid medium which does not contain any of such constituents (s) to (z) at all. As a result, the production of naphthoquinone derivatives in the cells may be further increased.

(h) The total concentration of cytokinins is decreased. That is, the total concentration of cytokinins in the medium used in the first stage is maintained between 0.01 μM and 10 μM, and the

second liquid medium contains cytokinins in a concentration of not more than 1/100 of the respective concentration in the first medium. The cytokinins used in the present invention may include 6-benzyladenine, 6-(3-methyl-2-butenylamino)purine (2ip), zeatin, dihydrozeatin and the like, besides kinetin.

(i) The concentration of sulfate ions is increased. That is, the concentration of sulfate ions in the medium used in the first stage amounts 0.5 to 3 mM, and the second liquid medium contains sulfate ions in at least ten times concentration of the respective concentration in the first medium.

The concentrations of other constituents than those the concentration thereof is substantially varied in the second stage according to the above stated provisions (a) to (i) may be varied in a wide range. In general, the inorganic components are adjusted within a range of about 0.1 µM to 200 mM, carbon sources within about 1 g/liter to 30 g/liter, phytohormones within about 0.01 µM to 30 µM, vitamins within about 1 mg/liter to 500 mg/liter, and amino acids within about 0.1 mg/liter to 100 mg/liter. Besides, in the present invention, it is also possible to further increase the naphthoquinone derivatives by compositely adjusting the constituents in the medium of the second stage by suitable combining several of the six embodiments (d) to (i). Particularly favourable is the combination of all the concentration modifications (a) to (i) in the second liquid medium.

Generally the rate of cell growth in the liquid medium (B) in the second stage is relatively lower than said cell growth in the liquid medium (A) in the first stage, but the production rate of the secondary metabolites such as naphthoquinone derivatives is significantly increased in the second liquid medium (B).

The initial concentration of cells in the liquid medium (B) may be varied in a wide range as in the case of the liquid medium (A). The period of time for the second stage cultures is 10 to 28 days, preferable 14 to 21 days.

The plant cells used in the present invention are callus or a mass of undifferential cells, prepared by taking a tissue sample from a plant body, such as roots, growing point, leaves, stems and seeds, and sterilizing it, adding it to a solid medium, and proliferating a part of the tissue cells to a mass of undifferentiated cells or callus.

The species of plants applied are not particularly limited, but may include, among others, *Juglandaceae* plants, such as chestnut, *Boraginaceae* plants such as *L. erythrorhizon*, *Pyrolaceae* plants such as *Pyrola incarnata* and *Chimaphila japonica*, *Plumbaginaceae* plants such as *Plumbago*, *Lythraceae* plants such as *Lawsonia inermis*, *Ranunculaceae* plants such as *Coptis japonica*, and *Solanaceae* plants such as *Nicotinia tabacum*.

In case of *Boraginaceae* plants, for example, tissue pieces of a plant body are sterilized, and placed on a Linsmaier-Skoog solid medium and cultured for about 7 to 30 days at 10 to 35°C in order to convert a part of the tissue pieces to callus. When the thus obtained callus is cultured for generation, the growth rate is gradually accelerated, and stabilized calluses are obtained. These calluses are applied to the liquid medium (A) for the first stage culturing.

The initial concentration of the callus in the liquid medium (A) may be varied in a wide range. Usually, it is preferred to add about 1 to 200 g (as a fresh weight) of callus in 1 liter of liquid medium.

In the first stage, the liquid medium (A) is preferably suited to promote the growth or the proliferation of callus. Even more preferred, the growth rate of the callus is accelerated by successive culturing in the liquid medium (A) beforehand.

In case of *Boraginaceae* plants, the secondary metabolites comprises natphthoquinone derivatives which may be produced in some extent even in the first stage culturing, but the amount thereof is slight. The condition suited to the proliferation of callus may not be neccessarily be good for the production of the secondary metabolites.

In the present invention, it is particularly important to encourage at the first stage culturing the proliferation of cells, and when the cells having such history are used, the production of the secondary metabolites such as naphthoquinone derivatives can be further increased in the second stage culturing in the modified liquid medium (referred to as "liquid medium (B)").

In case of *L. erythrorhizon* it is advantageful to merely proliferate callus in the first stage, usually 4 to 21 days, without encouraging production of the naphthoquinone derivatives, and then to proceed the second stage culturing in the different, second liquid medium usually for a period of 7 to 12 days.

The present invention provides favourable effects in particular when *Boraginaceae* plants are used to obtain shikonin and other naphthoquinone derivatives as the secondary metabolites.

However, the application of the invention is not limited to *L. erythrorhizon,* but the advantages of the present invention may be equally obtained for example in order to produce berberine from *Coptis japonica* or to produce nicotine from *Nictotinia tabacum.* Specifically in these latter cases, the effect of the present invention will be magnified by increasing the copper ion concentration in the second liquid medium as stated above as embodiment (d).

In case of *Boraginaceae* plants, cell culturing do not necessarily require light, and a dark place is rather preferred for the production of the secondary metabolites such as shikonin. The culturing temperature may be within a range of about 10°C to about 35°C, preferably within 23°C to 28°C approximately. At a temperature lower than 10°C the cell proliferation rate is low, and when the temperature exceeds 35°C, the proliferation rate is also decreased.

Naphthoquinone derivatives may be separated from the cells and the liquid medium (B), for example, by the conventional extraction method to obtain natural "Shikon" products.

According to the present invention, secondary metabolites may be efficiently produced from cells of higher plants. Since liquid media are used, upgrading of the scale or continuous operation would be easy, and the secondary metabolites can be produced in large quantities by simple operations.

The present invention is not limited to the culturing in two stages by using two different kinds of liquid media, but it is possible to culture the cells in three stages or more by using three or more kinds of liquid media having different composition, so far as the conditions of the present invention are satisfied.

The following working examples are intended for further explanation of the present invention.

Examples 1 and 2 and Comparative Examples 1 and 2

(Culturing in the first stage)

Thirty milliliters of a modified Linsmaier-Skoog liquid medium ($A_1$), having a composition as shown in Table 1 (containing 1 µM of indoleacetic acid, 10 µM of kinetin, and 30 g/liter of sucrose) are introduced into a 100 ml Erlenmeyer flask, and sterilized for 10 minutes at 120°C.

After being cooled, 0.5 g of a fresh callus of *L. erythrorhizon* (obtained by culture on the agar medium) are added to the above medium ($A_1$), and the whole composition is cultured at 25°C for a period of 14 days on a rotary shaker at an agitation diameter of 25 mm and at a rotation speed of 100 rpm.

The same operation is repeated (usually twice), using 0.5 g of the cells obtained by above mentioned culturing. As a result, the growth of cells in the modified liquid medium ($A_1$) is stabilized.

(Culturing in the second stage)

In conducting the second stage the above mentioned medium ($A_1$) or a medium ($C_1$, $B_1$ or $B_2$) as shown in Table 1 is used. The medium ($C_1$) according to a comparative example has been obtained from the medium ($A_1$) by decreasing the concentrations of each of the constituents (a) to (c) as mentioned above, but non of the concentration of the constituents (d) to (i) as mentioned above has been changed. The media $B_1$ and $B_2$ according to inventive examples have been obtlained from the medium ($A_1$) by decreasing the concentration of each of the constituents (a) to (c) as mentioned above; in addition the concentration of at least one of the constituents (d) to (i) as mentioned above has been changed.

In detail, 0.5 g of wet callus as obtained in the first stage culturing are added to 30 ml of the medium ($C_1$, $B_1$, $B_2$ or $A_1$), and the whole composition is cultured on a rotary shaker in the same manner as in the first stage.

After 14 days culturing the callus of *L. erythrorhizon* is separated by filtration, and dried for 24 hours at 35°C and thereafter is weighed in dry state. Thus, the dry weight of cultured cells per 1 liter of the liquid medium ($C_1$, $B_1$, $B_2$ or $A_1$) is determined.

Shikonin is extracted from the obtained cells, and is weighed to determine the total shikonin production per 1 liter of the liquid medium ($C_1$, $B_1$, $B_2$ or $A_1$).

The results obtained according to comparative examples 1 and 2, and according to inventive examples 1 and 2 are shown in Table 2.

Examples 3 to 23 and Comparative Examples 3 to 13

(Culturing in the first stage)

Thirty milliliters of a modified Linsmaier-Skoog liquid medium ($A_2$) having a composition as shown in Table 3 (containing 1 µM of indoleacetic acid, 10 µM of kinetin, and 30 g/liter of sucrose) are introduced into a 100 ml Erlenmeyer flask and sterilized for 10 minutes at 120°C.

After being cooled, 0.5 g of fresh callus of *L. erythrorhizon* (obtained by culture on the agar medium) are added to the above medium ($A_2$), and the whole composition is cultured at 25°C for a period of 14 days on a rotary shaker at an agitation diameter of 25 mm and at a rotation speed of 100 rpm.

The same operation is repeated (usually twice), using 0.5 g of the cells obtained by the above mentioned culture. As a result, the growth of cells in the modified liquid medium ($A_2$) is stabilized.

(Culturing in the second stage)

In conducting the second stage the above medium ($A_2$) or a medium ($C_2$ to $C_{11}$) or a medium ($B_3$ to $B_{23}$) as shown in Table 3 has been used. The media ($C_2$ to $C_{11}$) according to the comparative examples have been obtained from the medium ($A_2$) by the following modifications:

— medium ($C_2$): solely the inorganic nitrogen content has been decreased (and chloride content has been increased);

— medium ($C_3$): solely the inorganic nitrogen content has been decreased (and chloride content has been increased);

— medium ($C_4$): solely the inorganic phosphorus content has been decreased;

— medium ($C_5$): solely the inorganic phosphorus content has been decreased;

— medium ($C_6$): solely the potassium content has been decreased;

— medium ($C_7$): solely the potassium content has been decreased;

— medium ($C_8$): the contents of inorganic nitrogen, of potassium and of inorganic phosphorus have been decreased, but non of the constituents (d) to (i) as mentioned has been decreased;

— medium ($C_9$): the contents of inorganic nitrogen, of potassium and of inorganic phosphorus have been decreased, but non of the constituents (d) to (i) as mentioned has been decreased;

— medium ($C_{10}$): solely the copper content has been increased, but non of the constituents (a) to (c) as mentioned above has been decreased;

— medium ($C_{11}$): solely the copper content has been increased, but non of the constituents (a) to

(c) as mentioned above has been decreased;

The media ($B_3$) to ($B_{23}$) according to inventive examples have been obtained from medium ($A_2$) by decreasing the concentration of each of the constituents (a) to (c) as mentioned above and by changing the concentration of at least one of the constituents (d) to (i) as mentioned above.

In detail, 0.5 g of wet callus obtained in the first stage culturing are added to 30 ml of the medium ($C_2$ to $C_{11}$) or to the medium ($B_3$ to $B_{23}$) or to the medium ($A_2$), and the whole composition is cultured on a rotary shaker in the same manner as in the first stage culturing.

After 14 days culture, the callus of *L. erythrorhizon* is separated by filtration, and dried for 24 hours at 35°C and thereafter is weighed in dry stage. Thus, the dry weight of cultured cells per 1 liter of the liquid medium ($C_2$ to $C_{11}$ or $B_3$ to $B_{23}$ or $A_2$) was determined.

Shikonin is extracted from the obtained callus, and is weighed to determine the total shikonin production per 1 liter of the liquid medium ($C_2$ to $C_{11}$ or $B_3$ to $B_{23}$ or $A_2$).

The results obtained are shown in Table 4.

Examples 24 and 25, and Comparative Examples 14 and 15

(Culturing in the first stage)

Thirty milliliters of a modified Linsmaier-Skoog liquid medium ($A_3$ or $A_4$) having a composition as shown in Table 5 are introduced into a 100 ml Erlenmeyer flask, and sterilized for 10 minutes at 120°C.

After being cooled, 0.5 g of fresh callus of *Coptis japonica* or *Nicotiania tabacum* (obtained by culture on the agar medium) are added to the above medium ($A_3$ or $A_4$), and the whole composition is cultured at 25°C for a period of 14 days on a rotary shaker at an agitation diameter of 25 mm and at a rotation speed of 100 rpm.

The same operation is repeated (usually twice), using 0.5 g of the cells obtained by above mentioned culturing. As a result, the growth of cells in the modified liquid medium ($A_3$ or $A_4$) is stabilized.

(Culturing in the second stage)

In conducting the second stage, the media ($B_{24}$ or $B_{25}$) as shown in Table 5 have been prepared. In preparing the media ($B_{24}$) and ($B_{25}$) from the media ($A_3$ or $A_4$) the concentration of each of the constituents (a) to (c) as mentioned above has been decreased; in addition the concentration of at least one of the constituents (d) to (i) as mentioned above has been changed.

In the comparative examples 14 and 15, the same medium ($A_3$) or ($A_4$) respectively has been used in the first stage culturing and in the second stage culturing.

In detail, 0.5 g of wet callus as obtained in the first stage culturing are added to 30 ml of the medium ($B_{24}$ or $B_{25}$), and the whole composition is cultured on a rotary shaker in the same manner as in the first stage.

After 14 days culturing, the callus of *Coptis japonica* or *Nicotinia tabacam* is separated by filtration, and dried for 24 hours at 35°C and thereafter is weighed in dry state. Thus, the dry weight of cultured cells per 1 liter of the liquid medium ($B_{24}$ or $B_{25}$) is determined.

Berberine or nicotine is extracted from the obtained callus, and is weighed to determine the total production of berberine or nicotine per 1 liter of the liquid medium ($B_{24}$ or $B_{25}$).

The results obtained are shown in Table 6.

TABLE 1

| Class | Constituents | | $A_1$ | $C_1$ | $B_1$ | $B_2$ |
|---|---|---|---|---|---|---|
| Inorganic substances | $NH_4NO_3$ | (mM | 21 | — | — | — |
| | $KNO_3$ | (mM) | 19 | 1.9 | 1.9 | 1.9 |
| | $CaCl_2 . 2H_2O$ | (mM) | 3.0 | 3.0 | 3.0 | 3.0 |
| | $MgSO_4 . 7H_2O$ | (mM) | 3.0 | 3.0 | 3.0 | 3.0 |
| | $KH_2PO_4$ | (mM) | 1.25 | 0.125 | 0.125 | 0.125 |
| | KI | (µM) | 5 | 5 | — | — |
| | $H_3BO_3$ | (µM) | 100 | 100 | 100 | 100 |
| | $MnSO_4 . 4H_2O$ | (µM) | 100 | 100 | — | — |
| | $ZnSO_4 . 7H_2O$ | (µM) | 30 | 30 | 30 | 30 |
| | $Na_2MoO_4 . 2H_2O$ | (µM) | 1 | 1 | — | — |
| | $CuSO_4 . 5H_2O$ | (µM) | 0.6 | 0.6 | 0.6 | 0.6 |
| | $CoCl_2 . 6H_2O$ | (µM) | 0.1 | 0.1 | — | — |
| | $Na_3EDTA$ | (µM) | 100 | 100 | 10 | 10 |
| | $FeSO_4 . 7H_2O$ | (µM) | 100 | 100 | 10 | 10 |
| Carbon source | Sucrose | (g/l) | 30 | 30 | 30 | 30 |
| Vitamins | Inositol | (mg/l) | 100 | 100 | 100 | — |
| | Thiamine | (mg/l) | 0.4 | 0.4 | 0.4 | — |
| Amino acids | Glycine | (mg/l) | 0.2 | 0.2 | 0.2 | — |
| Phyto-hormones | IAA | (µM) | 1 | 1 | 1 | 1 |
| | Kinetin | (µM) | 10 | 10 | 10 | — |

(Note) $Na_3EDTA$: trisodium salt of ethylenediaminetetraacetic acid
IAA: indoleacetic acid

TABLE 2

| Examples | | Comp. Ex. 1 | Comp. Ex. 2 | Ex. 1 | Ex. 2 |
|---|---|---|---|---|---|
| first stage/ second stage | | $A_1/A_1$ | $A_1/C_1$ | $A_1/B_1$ | $A_1/B_2$ |
| Products | Callus (g/l) | 20.0 | 9.0 | 9.3 | 10.0 |
| | Shikonin (mg/l) | <100 | 740 | 970 | 1300 |

TABLE 3—1

| Class | Constituents | | $A_2$ | $C_2$ | $C_3$ | $C_4$ |
|---|---|---|---|---|---|---|
| Inorganic substances | $NH_4NO_3$ | (mM) | 21 | 3.8 | 2.1 | 21 |
| | $KNO_3$ | (mM) | 19 | 3.5 | 1.9 | 19 |
| | $NaNO_3$ | (mM) | — | — | — | — |
| | $CaCl_2 . 2H_2O$ | (mM) | 3.0 | 3.0 | 3.0 | 3.0 |
| | $MgSO_4 . 7H_2O$ | (mM) | 3.0 | 3.0 | 3.0 | 3.0 |
| | $KH_2PO_4$ | (mM) | 1.25 | 1.25 | 1.25 | 0.22 |
| | KI | (μM) | 5 | 5 | 5 | 5 |
| | $H_3BO_3$ | (μM) | 100 | 100 | 100 | 100 |
| | $MnSO_4 . 4H_2O$ | (μM) | 100 | 100 | 100 | 100 |
| | $ZnSO_4 . 7H_2O$ | (μM) | 30 | .30 | 30 | 30 |
| | $Na_2MoO_4 . 2H_2O$ | (μM) | 1 | 1 | 1 | 1 |
| | $CuSO_4 . 5H_2O$ | (μM) | 0.1 | 0.1 | 0.1 | 0.1 |
| | $CoCl_2 . 6H_2O$ | (μM) | 0.1 | 0.1 | 0.1 | 0.1 |
| | $Na_3EDTA$ | (μM) | 100 | 100 | 100 | 100 |
| | $FeSO_4 . 7H_2O$ | (μM) | 100 | 100 | 100 | 100 |
| | KCl | (mM) | — | 15.3 | 16.9 | — |
| Carbon source | Sucrose | (g/l) | 30 | 30 | 30 | 30 |
| Vitamins | Inositol | (mg/l) | 100 | 100 | 100 | 100 |
| | Thiamine | (mg/l) | 0.4 | 0.4 | 0.4 | 0.4 |
| Amino-acids | Glycine | (mg/l) | 2 | 2 | 2 | 2 |
| | L-glutamine | (mg/l) | 2 | 2 | 2 | 2 |
| Phyto-hormones | IAA | (μM) | 1.0 | 1.0 | 1.0 | 1.0 |
| | Kinetin | (μM) | 10 | 10 | 10 | 10 |

TABLE 3—2

| Class | Constituents | | $C_5$ | $C_6$ | $C_7$ | $C_8$ | $C_9$ |
|---|---|---|---|---|---|---|---|
| Inorganic substances | $NH_4NO_3$ | (mM) | 21 | 21 | 21 | 3.8 | 2.1 |
| | $KNO_3$ | (mM) | 19 | 3.5 | 1.9 | 3.5 | 1.9 |
| | $NaNO_3$ | (mM) | — | 15.3 | 16.9 | — | — |
| | $CaCl_2 . 2H_2O$ | (mM) | 3.0 | 3.0 | 3.0 | 3.0 | 3.0 |
| | $MgSo_4 . 7H_2O$ | (mM) | 3.0 | 3.0 | 3.0 | 3.0 | 3.0 |
| | $KH_2PO_4$ | (mM) | 0.125 | 1.25 | 1.25 | 0.22 | 0.125 |
| | KI | (µM) | 5 | 5 | 5 | 5 | 5 |
| | $H_3BO_3$ | (µM) | 100 | 100 | 100 | 100 | 100 |
| | $MnSO_4 . 4H_2O$ | (µM) | 100 | 100 | 100 | 100 | 100 |
| | $ZnSO_4 . 7H_2O$ | (µM) | 30 | 30 | 30 | 30 | 30 |
| | $Na_2MoO_4 . 2H_2O$ | (µM) | 1 | 1 | 1 | 1 | 1 |
| | $CuSO_4 . 5H_2O$ | (µM) | 0.1 | 0.1 | 0.1 | 0.1 | 0.1 |
| | $CoCl_2 . 6H_2O$ | (µM) | 0.1 | 0.1 | 0.1 | 0.1 | 0.1 |
| | $Na_3EDTA$ | (µM) | 100 | 100 | 100 | 100 | 100 |
| | $FeSO_4 . 7H_2O$ | (µM) | 100 | 100 | 100 | 100 | 100 |
| | KCl | (mM) | — | — | — | — | — |
| Carbon source | Sucrose | (g/l) | 30 | 30 | 30 | 30 | 30 |
| Vitamins | Inositol | (mg/l) | 100 | 100 | 100 | 100 | 100 |
| | Thiamine | (mg/l) | 0.4 | 0.4 | 0.4 | 0.4 | 0.4 |
| Amino acids | Glycine | (mg/l) | 2 | 2 | 2 | 2 | 2 |
| | L-glutamine | (mg/l) | 2 | 2 | 2 | 2 | 2 |
| Phyto-hormones | IAA | (µM) | 1.0 | 1.0 | 1.0 | 1.0 | 1.0 |
| | Kinetin | (µM) | 10 | 10 | 10 | 10 | 10 |

TABLE 3—3

| Class | Constituents | | $B_3$ | $B_4$ | $C_{10}$ | $C_{11}$ | $B_5$ |
|---|---|---|---|---|---|---|---|
| Inorganic substances | $NH_4NO_3$ | (mM) | 2.1 | 2.1 | 21 | 21 | 3.8 |
| | $KNO_3$ | (mM) | 1.9 | 1.9 | 19 | 19 | 3.5 |
| | $NaNO_3$ | (mM) | — | — | — | — | — |
| | $CaCl_2 . 2H_2O$ | (mM) | 3.0 | 3.0 | 3.0 | 3.0 | 3.0 |
| | $MgSO_4 . 7H_2O$ | (mM) | 3.0 | 3.0 | 3.0 | 3.0 | 3.0 |
| | $KH_2PO_4$ | (mM) | 0.125 | 0.125 | 1.25 | 1.25 | 0.22 |
| | KI | (µM) | 5 | 5 | 5 | 5 | 5 |
| | $H_3BO_3$ | (µM) | 100 | 100 | 100 | 100 | 100 |
| | $MnSO_4 . 4H_2O$ | (µM) | 100 | 100 | 100 | 100 | 100 |
| | $ZnSO_4 . 7H_2O$ | (µM) | 30 | 30 | 30 | 30 | 30 |
| | $Na_2MoO_4 . 2H_2O$ | (µM) | 1 | 1 | 1 | 1 | 1 |
| | $CuSO_4 . 5H_2O$ | (µM) | 0.3 | 1.2 | 0.3 | 1.2 | 0.3 |
| | $CoCl_2 . 6H_2O$ | (µM) | 0.1 | 0.1 | 0.1 | 0.1 | 0.1 |
| | $Na_3EDTA$ | (µM) | 100 | 100 | 100 | 100 | 100 |
| | $FeSO_4 . 7H_2O$ | (µM) | 100 | 100 | 100 | 100 | 100 |
| | KCl | (mM) | — | — | — | — | — |
| Carbon source | Sucrose | (g/l) | 30 | 30 | 30 | 30 | 30 |
| Vitamins | Inositol | (mg/l) | 100 | 100 | 100 | 100 | 100 |
| | Thiamine | (mg/l) | 0.4 | 0.4 | 0.4 | 0.4 | 0.4 |
| Amino acids | Glycine | (mg/l) | 2 | 2 | 2 | 2 | 2 |
| | L-glutamine | (mg/l) | 2 | 2 | 2 | 2 | 2 |
| Phyto-hormones | IAA | (µM) | 1.0 | 1.0 | 1.0 | 1.0 | 1.0 |
| | Kinetin | (µM) | 10 | 10 | 10 | 10 | 10 |

TABLE 3—4

| Class | Constituents | | $B_6$ | $B_7$ | $B_8$ | $B_9$ | $B_{10}$ |
|---|---|---|---|---|---|---|---|
| Inorganic substances | $NH_4NO_3$ | (mM) | 3.8 | 2.1 | 2.1 | 2.1 | 2.1 |
| | $KNO_3$ | (mM) | 3.5 | 1.9 | 1.9 | 1.9 | 1.9 |
| | $NaNO_3$ | (mM) | — | — | — | — | — |
| | $CaCl_2 . 2H_2O$ | (mM) | 3.0 | 3.0 | 3.0 | 3.0 | 3.0 |
| | $MgSO_4 . 7H_2O$ | (mM) | 3.0 | 3.0 | 3.0 | 3.0 | 3.0 |
| | $KH_2PO_4$ | (mM) | 0.22 | 0.125 | 0.125 | 0.125 | 0.125 |
| | KI | (μM) | 5 | 5 | 5 | 5 | 0.5 |
| | $H_3BO_3$ | (μM) | 100 | 100 | 100 | 100 | 100 |
| | $MnSO_4 . 4H_2O$ | (μM) | 100 | 100 | 9.0 | — | 100 |
| | $ZnSO_4 . 7H_2O$ | (μM) | 30 | 30 | 30 | 30 | 30 |
| | $Na_2MoO_4 . 2H_2O$ | (μM) | 1 | 1 | 1 | 1 | 1 |
| | $CuSO_4 . 5H_2O$ | (μM) | 1.2 | 2.4 | 1.2 | 1.2 | 1.2 |
| | $CoCl_2 . 6H_2O$ | (μM) | 0.1 | 0.1 | 0.1 | 0.1 | 0.1 |
| | $Na_3EDTA$ | (μM) | 100 | 100 | 100 | 100 | 100 |
| | $FeSO_4 . 7H_2O$ | (μM) | 100 | 100 | 100 | 100 | 100 |
| | KCl | (mM) | — | — | — | — | — |
| Carbon source | Sucrose | (g/l) | 30 | 30 | 30 | 30 | 30 |
| Vitamins | Inositol | (mg/l) | 100 | 100 | 100 | 100 | 100 |
| | Thiamine | (mg/l) | 0.4 | 0.4 | 0.4 | 0.4 | 0.4 |
| Amino acids | Glycine | (mg/l) | 2 | 2 | 2 | 2 | 2 |
| | L-glutamine | (mg/l) | 2 | 2 | 2 | 2 | 2 |
| Phyto-hormones | IAA | (μM) | 1.0 | 1.0 | 1.0 | 1.0 | 1.0 |
| | Kinetin | (μM) | 10 | 10 | 10 | 10 | 10 |

TABLE 3—5

| Class | Constituents | | $B_{11}$ | $B_{12}$ | $B_{13}$ | $B_{14}$ | $B_{15}$ |
|---|---|---|---|---|---|---|---|
| Inorganic substances | $NH_4NO_3$ | (mM) | 2.1 | 2.1 | 2.1 | 2.1 | 2.1 |
| | $KNO_3$ | (mM) | 1.9 | 1.9 | 1.9 | 1.9 | 1.9 |
| | $NaNO_3$ | (mM) | — | — | — | — | — |
| | $CaCl_2 . 2H_2O$ | (mM) | 3.0 | 3.0 | 3.0 | 3.0 | 3.0 |
| | $MgSO_4 . 7H_2O$ | (mM) | 3.0 | 3.0 | 3.0 | 3.0 | 3.0 |
| | $KH_2PO_4$ | (mM) | 0.125 | 0.125 | 0.125 | 0.125 | 0.125 |
| | KI | (μM) | — | 5 | 5 | 5 | — |
| | $H_3BO_3$ | (μM) | 100 | 100 | 100 | 100 | 100 |
| | $MnSO_4 . 4H_2O$ | (μM) | 100 | 100 | 100 | 9 | — |
| | $ZnSO_4 . 7H_2O$ | (μM) | 30 | 30 | 30 | 30 | 30 |
| | $Na_2MoO_4 . 2H_2O$ | (μM) | 1 | 0.08 | — | 0.08 | — |
| | $CuSO_4 . 5H_2O$ | (μM) | 1.2 | 1.2 | 1.2 | 1.2 | 1.2 |
| | $CoCl_2 . 6H_2O$ | (μM) | 0.1 | 0.1 | 0.1 | 0.1 | 0.1 |
| | $Na_3EDTA$ | (μM) | 100 | 100 | 100 | 100 | 100 |
| | $FeSO_4 . 7H_2O$ | (μM) | 100 | 100 | 100 | 100 | 100 |
| | KCl | (mM) | — | — | — | — | — |
| Carbon source | Sucrose | (g/l) | 30 | 30 | 30 | 30 | 30 |
| Vitamins | Inositol | (mg/l) | 100 | 100 | 100 | 100 | 100 |
| | Thiamine | (mg/l) | 0.4 | 0.4 | 0.4 | 0.4 | 0.4 |
| Amino acids | Glycine | (mg/l) | 2 | 2 | 2 | 2 | 2 |
| | L-glutamine | (mg/l) | 2 | 2 | 2 | 2 | 2 |
| Phyto-hormones | IAA | (μM) | 1.0 | 1.0 | 1.0 | 1.0 | 1.0 |
| | Kinetin | (μM) | 10 | 10 | 10 | 10 | 10 |

TABLE 3—6

| Class | Constituents | | $B_{16}$ | $B_{17}$ | $B_{18}$ | $B_{19}$ | $B_{20}$ |
|---|---|---|---|---|---|---|---|
| Inorganic substances | $NH_4NO_3$ | (mM) | 2.1 | 2.1 | 2.1 | 2.1 | 2.1 |
| | $KNO_3$ | (mM) | 1.9 | 1.9 | 1.9 | 1.9 | 1.9 |
| | $NaNO_3$ | (mM) | — | — | — | — | — |
| | $CaCl_2 . 2H_2O$ | (mM) | 3.0 | 3.0 | 3.0 | 3.0 | 3.0 |
| | $MgSO_4 . 7H_2O$ | (mM) | 3.0 | 3.0 | 3.0 | 3.0 | 3.0 |
| | $KH_2PO_4$ | (mM) | 0.125 | 0.125 | 0.125 | 0.125 | 0.125 |
| | KI | (μM) | 5 | 5 | 5 | 5 | 5 |
| | $H_3BO_3$ | (μM) | 100 | 100 | 100 | 100 | 100 |
| | $MnSO_4 . 4H_2O$ | (μM) | 100 | 100 | 100 | 100 | 100 |
| | $ZnSO_4 . 7H_2O$ | (μM) | 30 | 30 | 30 | 30 | 30 |
| | $Na_2MoO_4 . 2H_2O$ | (μM) | 1 | 1 | 1 | 1 | 1 |
| | $CuSO_4 . 5H_2O$ | (μM) | 1.2 | 1.2 | 1.2 | 1.2 | 1.2 |
| | $CoCl_2 . 6H_2O$ | (μM) | 0.1 | 0.1 | 0.1 | 0.1 | 0.1 |
| | $Na_3EDTA$ | (μM) | 100 | 100 | 100 | 100 | 100 |
| | $FeSO_4 . 7H_2O$ | (μM) | 100 | 100 | 100 | 100 | 100 |
| | KCl | (mM) | — | — | — | — | — |
| Carbon source | Sucrose | (g/l) | 30 | 30 | 30 | 30 | 30 |
| Vitamins | Inositol | (mg/l) | 8 | — | 100 | 100 | 100 |
| | Thiamine | (mg/l) | 0.03 | — | 0.4 | 0.4 | 0.4 |
| Amino acids | Glycine | (mg/l) | 2 | 2 | 0.01 | — | 2 |
| | L-glutamine | (mg/l) | 2 | 2 | 0.01 | — | 2 |
| Phyto-hormones | IAA | (μM) | 1.0 | 1.0 | 1.0 | 1.0 | 1.0 |
| | Kinetin | (μM) | 10 | 10 | 10 | 10 | 0.1 |

TABLE 3—7

| Class | Constituents | | $B_{21}$ | $B_{22}$ | $B_{23}$ |
|---|---|---|---|---|---|
| Inorganic substances | $NH_4NO_3$ | (mM | 2.1 | 2.1 | 2.1 |
| | $KNO_3$ | (mM) | 1.9 | 1.9 | 1.9 |
| | $NaNO_3$ | (mM) | — | — | — |
| | $CaCl_2 . 2H_2O$ | (mM) | 3.0 | 3.0 | 3.0 |
| | $MgSO_4 . 7H_2O$ | (mM) | 3.0 | 3.0 | 3.0 |
| | $KH_2PO_4$ | (mM) | 0.125 | 0.125 | 0.125 |
| | KI | (μM) | 5 | 0.08 | — |
| | $H_3BO_3$ | (μM) | 100 | 100 | 100 |
| | $MnSO_4 . 4H_2O$ | (μM) | 100 | 2.0 | — |
| | $ZnSO_4 . 7H_2O$ | (μM) | 30 | 30 | 30 |
| | $Na_2MoO_4 . 2H_2O$ | (μM) | 1 | 0.02 | — |
| | $CuSO_4 . 5H_2O$ | (μM) | 1.2 | 0.08 | 0.3 |
| | $CoCl_2 . 6H_2O$ | (μM) | 0.1 | 0.1 | 0.1 |
| | $Na_3EDTA$ | (μM) | 100 | 100 | 100 |
| | $FeSO_4 . 7H_2O$ | (μM) | 100 | 100 | 100 |
| | KCl | (mM) | — | — | — |
| Carbon source | Sucrose | (g/l) | 30 | 30 | 30 |
| Vitamins | Inositol | (mg/l) | 100 | 8 | — |
| | Thiamine | (mg/l) | 0.4 | 0.03 | — |
| Amino acids | Glycine | (mg/l) | 2 | 0.01 | — |
| | L-glutamine | (mg/l) | 2 | 0.01 | — |
| Phyto-hormones | IAA | (μM) | 1.0 | 1.0 | 1.0 |
| | Kinetin | (μM) | — | 0.1 | — |

TABLE 4—1

| Examples | | Comp. Ex. 3 | Comp. Ex. 4 | Comp. Ex. 5 | Comp. Ex. 6 | Comp. Ex. 7 | Comp. Ex. 8 | Comp. Ex. 9 |
|---|---|---|---|---|---|---|---|---|
| First stage/ Second stage | | $A_2/A_2$ | $A_2/C_2$ | $A_2/C_3$ | $A_2/C_4$ | $A_2/C_5$ | $A_2/C_6$ | $A_2/C_7$ |
| Products | Callus (g/l) | 20.0 | 19.0 | 14.2 | 16.5 | 7.7 | 18.1 | 17.0 |
| | Shikonin (mg/l) | <100 | 110 | 200 | 280 | 400 | 130 | 150 |

| Examples | | Comp. Ex. 10 | Comp. Ex. 11 | Ex. 3 | Ex. 4 | Comp. Ex. 12 | Comp. Ex. 13 | Ex. 5 |
|---|---|---|---|---|---|---|---|---|
| First stage/ Second stage | | $A_2/C_8$ | $A_2/C_9$ | $A_2/B_3$ | $A_2/B_4$ | $A_2/C_{10}$ | $A_2/C_{11}$ | $A_2/B_5$ |
| Products | Callus (g/l) | 12.2 | 8.3 | 8.5 | 8.6 | 20.4 | 20.6 | 12.4 |
| | Shikonin (mg/l) | 420 | 500 | 690 | 850 | 150 | 200 | 480 |

TABLE 4—2

| Examples | | Ex. 6 | Ex. 7 | Ex. 8 | Ex. 9 | Ex. 10 | Ex. 11 | Ex. 12 |
|---|---|---|---|---|---|---|---|---|
| First stage/ Second stage | | $A_2/B_6$ | $A_2/B_7$ | $A_2/B_8$ | $A_2/B_9$ | $A_2/B_{10}$ | $A_2/B_{11}$ | $A_2/B_{12}$ |
| Products | Callus (g/l) | 13.1 | 8.3 | 8.7 | 8.7 | 8.5 | 8.8 | 8.3 |
| | Shikonin (mg/l) | 550 | 850 | 530 | 550 | 540 | 570 | 530 |

| Examples | | Ex. 13 | Ex. 14 | Ex. 15 | Ex. 16 | Ex. 17 | Ex. 18 | Ex. 19 |
|---|---|---|---|---|---|---|---|---|
| First stage/ Second stage | | $A_2/B_{13}$ | $A_2/B_{14}$ | $A_2/B_{15}$ | $A_2/B_{16}$ | $A_2/B_{17}$ | $A_2/B_{18}$ | $A_2/B_{19}$ |
| Products | Callus (g/l) | 8.3 | 8.9 | 9.1 | 9.2 | 9.5 | 9.0 | 9.2 |
| | Shikonin (mg/l) | 530 | 570 | 640 | 620 | 700 | 580 | 590 |

TABLE 4—3

| Example | | Ex. 20 | Ex. 21 | Ex. 22 | Ex. 23 |
|---|---|---|---|---|---|
| First stage/ Second stage | $A_2/B_{20}$ | $A_2/B_{21}$ | $A_2/B_{22}$ | $A_2/B_{23}$ | |
| Products | Callus (g/l) | 9.0 | 9.2 | 9.9 | 10.0 |
| | Shikonin (mg/l) | 690 | 700 | 1010 | 1300 |

TABLE 5

| Class | Constituents | | Coptis | | Nicotiana | |
|---|---|---|---|---|---|---|
| | | | $A_3$ | $B_{24}$ | $A_4$ | $B_{25}$ |
| Inorganic substances | $NH_4NO_3$ | (mM) | 18 | 2.1 | 18 | 2.1 |
| | $KNO_3$ | (mM) | 19 | 1.9 | 19 | 1.9 |
| | $CaCl_2 . 2H_2O$ | (mM) | 1.1 | 3.0 | 1.1 | 3.0 |
| | $MgSO_4 . 7H_2O$ | (mM) | 0.75 | 3.0 | 0.75 | 3.0 |
| | $KH_2PO_4$ | (mM) | 1.0 | 0.125 | 1.0 | 0.125 |
| | KI | (µM) | 5 | — | 5 | — |
| | $H_3BO_3$ | (µM) | 161 | 100 | 161 | 100 |
| | $MnSO_4 . 4H_2O$ | (µM) | 112 | — | 112 | — |
| | $ZnSO_4 . 7H_2O$ | (µM) | 35 | 30 | 35 | 30 |
| | $Na_2MoO_4 . 2H_2O$ | (µM) | 1 | — | 1 | — |
| | $CuSO_4 . 5H_2O$ | (µM) | 0.1 | 0.3 | 0.1 | 0.3 |
| | $Na_3EDTA$ | (µM) | 100 | 100 | 100 | 100 |
| | $FeSO_4 . 7H_2O$ | (µM) | 100 | 100 | 100 | 100 |
| Carbon source | Sucrose | (g/l) | 20 | 30 | 20 | 30 |
| Vitamins | Inositol | (mg/l) | 100 | — | 100 | — |
| | Thiamine | (mg/l) | 0.5 | — | 0.5 | — |
| Amino acids | Glycine | (mg/l) | 2 | — | 2 | — |
| Phyto-hormones | IAA | (µM) | — | 1.0 | 10 | 1.0 |
| | Kinetin | (µM) | — | — | 1 | — |
| | NAA | (µM) | 10 | — | — | — |
| | BA | (µM) | 0.007 | — | — | — |

TABLE 6

| Examples | Comp. Ex. 14 | Ex. 24 | Comp. Ex. 15 | Ex. 25 |
|---|---|---|---|---|
| First stage/ Second stage | $A_3/A_3$ | $A_3/B_{24}$ | $A_4/A_4$ | $A_4/B_{25}$ |
| Callus (g/l) | 10.0 | 7.5 | 13.2 | 1.4 |
| Products (mg/l) | berberine | berberine | nicotine | nicotine |
| | 170 | 300 | 1.4 | 2.3 |

**Claims**

1. A method for producing secondary metabolites of plants by culturing cells of said plants obtained by culturing in a first liquid medium, the composition thereof favours the growth of said plants and comprises inorganic substances and carbon sources as the essential constituents and further containing at least one of phytohormones, vitamins and amino acids, and further culturing said cells obtained from the first medium in a second liquid medium, the composition thereof favours the production of the secondary metabolites characterized in that the composition of the second liquid medium differs from the composition of the corresponding first liquid medium in at least two of the following concentrations (a) to (c), namely:

(a) the first medium contains inorganic nitrogen in an amount of 40 to 130 mM and the second medium contains inorganic nitrogen in a concentration of not more than 1/5 of the respective concentration in the first medium;

(b) the first medium contains inorganic phosphorus in an amount of 0.8 to 2.4 mM and the second medium contains inorganic phosphorus in a concentration of not more than 1/5 of the respective concentration in the first medium;

(c) the first medium contains potassium in an amount of 10 to 45 mM and the second medium contains potassium in a concentration of not more than 1/5 of the respective concentration in the first medium;

and concurrently in at least one of the concentrations (d) to (i), namely:

(d) the first medium contains copper ions in an amount of 0.015 to 0.15 µM, and the second medium contains copper ions in at least the three times concentration of the respective concentration in the first medium;

(e) the first medium contains manganese ions in an amount of 30 to 300 µM, iodine ions in an amount of 1 to 15 µM, and molybdate ions in an amount of 0.05 to 0.5 µM, and the second medium contains at least one of manganese ions, iodine ions and molybdate ions in a concentration of not more than 1/10 of the respective concentration in the first medium;

(f) the first medium contains vitamins in a total amount of 1 to 500 mg/liter, and the second medium contains vitamins in a concentration of not more than 1/10 of the respective concentration in the first medium;

(g) the first medium contains amino acids in a total amount of 0.1 to 100 mg/liter, and the second medium contains amino acids in a concentration of not more than 1/100 of the respective concentration in the first medium;

(h) the first medium contains cytokinins in a total amount of 0.01 to 10 µM, and the second medium contains cytokinins in a concentration of not more than 1/100 of the respective concentration in the first medium;

(i) the first medium contains sulfate ions in an amount of 0.5 to 3 mM, and the second medium contains sulfate ions in at least the ten times concentration of the respective concentration in the first medium.

2. The method according to claim 1, wherein the composition of the second liquid medium differs from the composition of the corresponding first liquid medium in all the concentrations (a) to (c).

3. The method according to claim 1 or 2, wherein naphthochinon derivates are produced from plants of the *Boraginaceae* family, *Juglandaceae* family or *Lythraceae* family.

4. The method according to claim 3, wherein the naphthochinon derivate is Shikonin and the plant is *Lithospermum erythrorhizon.*

5. The method according to claim 1 or 2, wherein isoquinoline alkaloids are produced from plants of the *Ranunculaceae* family.

6. The method according to claim 5, wherein the isoquinoline alkaloid is berberine and the plant is *Coptis japonica.*

7. The method according to claim 1 or 2, wherein the second metabolites are produced from plants of the *Solanaceae* family.

8. The method according to claim 7, wherein nicotine is produced from *Nicotiania tabacum.*

**Patentansprüche**

1. Ein Verfahren zur Produktion von sekundären Metaboliten von Pflanzen durch Vermehrung von Zellen dieser Pflanzen, die durch Vermehrung in

einer ersten Kulturbrühe erhalten worden sind, deren Zusammensetzung das Wachstum der Pflanzen begünstigt, und zu deren wesentlichen Bestandteilen anorganische Stoffe und Kohlenstoffquellen gehören, sowie Pflanzenhormone, Vitamine und/oder Aminosäuren, und die in der ersten Kulturbrühe erhaltenen Zellen daraufhin in einer zweiten Kulturbrühe vermehrt werden, deren Zusammensetzung die Produktion der sekundären Metaboliten begünstigt, dadurch gekennzeichnet, daß sich die Zusammensetzung der zweiten Kulturbrühe von der Zusammensetzung der ersten Kulturbrühe in wenigstens zwei der nachstehenden Bestandteilkonzentrationen (a) bis (c) unterscheidet, nämlich

(a) die erste Kulturbrühe enthält 40 bis 130 mM anorganischen Stickstoff, und die zweite Kulturbrühe enthält nicht mehr als 1/5 des Gehaltes der ersten Kulturbrühe an anorganischem Stickstoff;

(b) die erste Kulturbrühe enthält 0,8 bis 2,4 mM anorganischen Phosphor, und die zweite Kulturbrühe enthält nicht mehr als 1/5 des Gehaltes der ersten Kulturbrühe an organischem Phosphor;

(c) die erste Kulturbrühe enthält 10 bis 45 mM Kalium, und die zweite Kulturbrühe enthält nicht mehr als 1/5 des Gehaltes der ersten Kulturbrühe an Kalium;

und sich gleichzeitig die Zusammensetzung der ersten Kulturbrühe von der Zusammensetzung der zweiten Kulturbrühe in wenigstens einer der nachstehenden Bestandteilkonzentrationen (d) bis (i) unterscheidet, nämlich

(d) die erste Kulturbrühe enthält 0,015 bis 0,15 µM Kupferionen, und die zweite Kulturbrühe enthält wenigstens den dreifachen Gehalt der ersten Kulturbrühe an Kupferionen;

(e) die erste Kulturbrühe enthält 30 bis 300 µM Manganionen, 1 bis 15 µM Jodidionen, und 0,05 bis 0,5 µM Molybdationen, und die zweite Kulturbrühe enthält nicht mehr als 1/10 des Gehaltes der ersten Kulturbrühe an Manganionen, Jodidionen und/oder Molybdationen;

(f) die erste Kulturbrühe enthält Vitamine in einem Gesamtgehalt von 1 bis 500 mg/l, und die zweite Kulturbrühe enthält nicht mehr als 1/10 des Vitamingehaltes der ersten Kulturbrühe;

(g) die erste Kulturbrühe enthält Aminosäuren in einem Gesamtgehalt von 0,1 bis 100 mg/l, und die zweite Kulturbrühe enthält nicht mehr als 1/100 des Gehaltes der ersten Kulturbrühe an Aminosäuren;

(h) die erste Kulturbrühe enthält 0,01 bis 10 µM Cytokinine, und die zweite Kulturbrühe enthält nicht mehr als 1/100 des Gehaltes der ersten Kulturbrühe an Cytokininen;

(i) die erste Kulturbrühe enthält 0,5 bis 3 mM Sulfationen, und die zweite Kulturbrühe enthält wenigstens den 10-fachen Gehalt der ersten Kulturbrühe an Sulfationen.

2. Das Verfahren nach Anspruch 1, wobei sich die Zusammensetzung der zweiten Kulturbrühe von der Zusammensetzung der ersten Kulturbrühe in allen Bestandteilkonzentrationen (a) bis (c) unterscheidet.

3. Das Verfahren nach Anspruch 1 oder 2, wobei aus Pflanzen der Gattung *Boraginaceae* oder *Juglandaceae* oder *Lythraceae* Naphthochinonderivate erzeugt werden.

4. Das Verfahren nach Anspruch 3, wobei aus der Pflanze *Lithospermum Erythrorhizon* Shikonin als Naphthochinonderivat erzeugt wird.

5. Das Verfahren nach Anspruch 1 oder 2, wobei aus Pflanzen der Gattung *Ranunculaceae* Isochinolin-Alkaloide erzeugt werden.

6. Das Verfahren nach Anspruch 5, wobei aus der Pflanze *Coptis Japonica* Berberin als Isochinolin-Alkaloid erzeugt wird.

7. Das Verfahren nach Anspruch 1 oder 2, wobei aus Pflanzen der Gattung *Solanaceae* sekundäre Metaboliten erzeugt werden.

8. Das Verfahren nach Anspruch 7, wobei aus *Nicotiania Tabacum* Nikotin erzeugt wird.

## Revendications

1. Procédé de production de métabolites secondaires de plantes par culture de cellules desdites plantes obtenues par culture dans un premier milieu liquide, dont la composition favorise la croissance desdites plantes et comprend des substances minérales et des sources de carbone comme constituants essentiels et contenant en outre des phtyo-hormones, des vitamines et/ou des acides aminés, puis par culture desdites cellules obtenues à partir du premier milieu dans un second milieu liquide, dont la composition favorise la production des métabilites secondaires, caractérisé en ce que la composition du second milieu liquide diffère de la composition du premier milieu liquide correspondant par au moins deux des concentrations (a) à (c) suivantes, à savoir:

(a) le premier milieu contient de l'azote minéral dans une quantité de 40 à 130 mM et le second milieu contient de l'azote minéral à une concentration représentant pas plus de 1/5 de la concentration correspondante dans le premier milieu;

(b) le premier milieu contient du phosphore minéral dans une quantité de 0,8 à 2,4 mM et le second milieu contient du phosphore minéral à une concentration ne représentant pas plus de 1/5 de la concentration correspondante dans le premier milieu;

(c) le premier milieu contient du potassium dans une quantité de 10 à 45 mM et le second milieu contient du potassium à une concentration ne représentant pas plus de 1/5 de la concentration correspondante dans le premier milieu;

et en même temps par au moins une des concentrations (d) à (i) suivantes, à savoir:

(d) le premier milieu contient des ions cuivre dans une quantité de 0,015 à 0,15 µM, et le second milieu contient des ions cuivre à une concentration représentant au moins trois fois la concentration correspondante dans le premier milieu;

(e) le premier milieu contient des ions manganèse dans une quantité de 30 à 300 µM, des ions iode dans une quantité de 1 à 15 µM, et des ions

molybdate dans une quantité de 0,05 à 0,5 µM, et le second milieu contient des ions manganèse, des ions iode et/ou des ions molybdate à une concentration ne représentant pas plus de 1/10 de la concentration correspondante dans le premier milieu;

(f) le premier milieu contient des vitamines dans une quantité totale de 1 à 500 mg/litre, et le second milieu contient des vitamines à une concentration ne représentant pas plus de 1/10 de la concentration correspondante dans le premier milieu;

(g) le premier milieu contient des acides aminés dans une quantité totale de 0,1 à 100 mg/litre, et le second milieu contient des acides aminés à une concentration ne représentant pas plus de 1/100 de la concentration correspondante dans le premier milieu;

(h) le premier milieu contient des cytokinines dans une quantité totale de 0,01 à 10 µM, et le second milieu contient des cytokinines à une concentration ne représentant pas plus de 1/100 de la concentration correspondante dans le premier milieu;

(i) le premier milieu contient des ions sulfate dans une quantité de 0,5 à 3 mM, et le second milieu contient des ions sulfate à une concentration représentant au moins dix fois la concentration correspondante dans le premier milieu.

2. Procédé selon la revendication 1, dans lequel la composition du second milieu liquide diffère de la composition du premier milieu liquide correspondant par l'ensemble des concentrations (a) à (c).

3. Procédé selon la revendication 1 ou 2, dans lequel des dérivés de la naphtoquinone sont produits à partir de plantes de la famille des *Boraginaceae*, de la famille des *Juglandaceae* ou de la famille des *Lythraceae*.

4. Procédé selon la revendication 3, dans lequel le dérivé de la naphtoquinone est la shikonine et la plante est *Lithospermum erythrorhizon*.

5. Procédé selon la revendication 1 ou 2, dans lequel des alcaloïdes de l'isoquinoléine sont produits à partir de plantes de la famille des *Ranunculaceae*.

6. Procédé selon la revendication 5, dans lequel l'alcaloïde d'isoquinoléine est la berbérine et la plante est *Coptis japonica*.

7. Procédé selon la revendication 1 ou 2, dans lequel les métabolites secondaires sont produits à partir de plantes de la famille des *Solanaceae*.

8. Procédé selon la revendication 7, dans lequel de la nicotine est produite à partir de *Nicotiana tabacum*.